# EUROPÄISCHE PATENTSCHRIFT

(11) **EP 3 082 752 B1**
(45) Veröffentlichungstag und Bekanntmachung des Hinweises auf die Patenterteilung: **15.01.2020**
(21) Anmeldenummer: 14800104.3
(22) Anmeldetag: 21.11.2014
(51) Int. Cl.: A61K 8/49, A61Q 19/00, A61K 31/426, A61P 17/00

(54) **VERWENDUNG VON ALKYLAMIDOTHIAZOLEN IN KOSMETISCHEN ODER DERMATOLOGISCHEN ZUBEREITUNGEN ZUR PROPHYLAXE VOR UND BEHANDLUNG VON SENSIBLER HAUT**
USE OF ALKYLAMIDOTHIAZOLES IN COSMETIC OR DERMATOLOGICAL PREPARATIONS FOR THE PROPHYLAXIS OR TREATMENT OF SENSITIVE SKIN
UTILISATION D'ALKYLAMIDOTHIAZOLES DANS DES PRÉPARATIONS COSMÉTIQUES OU DERMATOLOGIQUES POUR ASSURER LA PROPHYLAXIE ET LE TRAITEMENT DES PEAUX SENSIBLES

(30) Priorität: 19.12.2013 DE 102013226711
(43) Veröffentlichungstag der Anmeldung: 26.10.2016
(73) Patentinhaber: Beiersdorf AG, 20253 Hamburg (DE)
(72) Erfinder: KOLBE, Ludger, 21255 Dohren (DE); SCHERNER, Cathrin, 22844 Norderstedt (DE); WENSORRA, Ursula, 21035 Hamburg (DE); MANN, Tobias, 22175 Hamburg (DE)
(86) Internationale Anmeldenummer: PCT/EP2014/075319
(87) Internationale Veröffentlichungsnummer: WO 2015/090851

(56) Entgegenhaltungen:
- WO-A1-2013/041526
- WO-A1-2013/041535
- WO-A1-2014/139757
- WO-A1-2014/139758
- WO-A1-2014/139759
- WO-A2-2011/117034

## Beschreibung

Die vorliegende Erfindung betrifft die Verwendung von Alkylamidothiazolen in kosmetischen oder dermatologischen Zubereitungen zur Prophylaxe vor und Behandlung von sensibler Haut, Juckreiz, sowie von entzündlichen Erscheinung in der menschlichen Haut.

Alterungsprozeß verbundene Veränderungen und extrinsische Faktoren (z.B. Schädigung der Hautbarriere, Einwirkung von UV-Licht, irritierenden oder allergie-auslösenden Substanzen, mechanische Einwirkung z.B. durch Rasur) können zu einer Hautirritation führen. Unter Hautirritation ist im Zusammenhang mit dieser Anmeldung jede Veränderung der Haut zu verstehen, die bei Mensch oder Tier sensorisches Missempfinden auslöst oder/und durch ein trockenes, gerötetes und/oder entzündetes Hautbild geprägt ist. Dabei umfasst der Begriff sensorisches Missempfinden selbstverständlich auch Zustände wie Jucken oder Schmerz. Hautirritation kann insbesondere phänomenologisch verschiedene Hautzustände umfassen: Sensible Haut, empfindliche Haut inklusive empfindlicher Kopfhaut, verletzliche Haut, trockene Haut, atopische und psoriatische Haut, irritierte Haut, Rosacea, entzündete Haut, sowie Hautveränderungen bei Diabetes mellitus, die sich im jeweils höheren Schweregrad in einer Hautrötung, dem sogenannten Erythem äußert.

Das Problem "sensible Haut" betrifft eine wachsende Anzahl Erwachsener und Kinder. Man geht inzwischen davon aus, dass bis zu 50 % der Bevölkerung eine sensible Haut haben (L. Misery et al., Ann. Dermatol. Venereol. 2005, 132, 425-429). Mit sensibler Haut bezeichnet man eine Haut mit einer erniedrigten Reizschwelle für Irritantien, wie hyperreaktive und intolerante, aber auch atopische Haut. Bei Menschen mit sensibler, empfindlicher oder verletzlicher Haut kann ein mit "Stinging" (engl. "to sting" = verletzen, brennen, schmerzen) bezeichnetes Phänomen beobachtet werden. Typische, mit den Begriffen "Stinging" oder "empfindlicher Haut" in Verbindung gebrachte, störende Phänomene sind Hautrötung, Kribbeln, Prickeln, Spannen und Brennen der Haut und Juckreiz. Sie können durch stimulierende Umgebungsbedingungen wie z. B. Massage, Tensideinwirkung, Einwirkung weiterer chemischer Substanzen wie z.B. Milchsäure, Wettereinfluss wie Wärme, Kälte, Trockenheit, aber auch feuchte Wärme, Wärmestrahlung und UV-Strahlung, z. B. der Sonne, oder psychologischen Stress hervorgerufen werden.

"Empfindliche" Kopfhaut ist ebenfalls gekennzeichnet durch Hautrötung, Kribbeln, Prickeln, Brennen und Juckreiz. Auslöser sind beispielsweise Seife, Shampoos oder andere Haarpflegemittel, Tenside, hartes Wasser mit hohen Kalkkonzentrationen und/oder mechanische Beanspruchung. Erytheme und Hyperseborrhoe (übermäßige Talgproduktion) der Kopfhaut sowie Schuppen sind häufig mit den beschriebenen Phänomenen assoziiert.

Bei ca. 10-20% der Bevölkerung industrieller Länder, mit steigender Tendenz, ist die Atopie zu beobachten, eine familiär auftretende Überempfindlichkeit der Haut und der Schleimhäute gegen Umweltstoffe, mit gesteigerter Bereitschaft, gegen Substanzen aus der natürlichen Umwelt Überempfindlichkeitsreaktionen vom Soforttyp (Allergien) zu entwickeln. Atopie ist vermutlich genetisch bedingt. Atopie kann sich als atopische Dermatitis äußern. Dabei ist die Hautbarriere geschädigt, die Haut ist oft entzündet und juckt.

Die erythematöse Wirkung des ultravioletten Teils der Sonnenstrahlung oder künstlicher Strahlung auf die Haut ist allgemein bekannt. Während Strahlen mit einer Wellenlänge, die kleiner als 290 nm ist (der sogenannte UVC-Bereich), von der Ozonschicht in der Erdatmosphäre absorbiert werden, verursachen Strahlen im Bereich zwischen 290 nm und 320 nm, dem sogenannten UVB-Bereich, ein Erythem, einen einfachen Sonnenbrand oder sogar mehr oder weniger starke Verbrennungen. Erythematöse Hauterscheinungen treten auch als Begleiterscheinungen bei gewissen Hauterkrankungen oder - unregelmässigkeiten auf, beispielsweise Akne, bakteriell induzierte Entzündungen der Haut, Kutanreaktionen, Dermographismus, das Wundsein der Haut im allgemeinen, Hautmaulwurf, Wundrose, Gürtelrose, Erfrierungen oder Verbrennungen. Eine besondere Form der Hautrötung stellt dabei die Rosacea dar. Antiinflammatorisch wirksame Zubereitungen können daher auch vorteilhaft eingesetzt werden allgemein krankheitsbedingte Hautrötungen und im speziellen die Rosacea abklingen zu lassen, über einen längeren Zeitraum zu unterdrücken und das wieder Auftreten weitgehend zu verhindern.

Hautentzündungen, die mit Rötung und Juckreiz einhergehen, werden ebenfalls durch Insektenstiche hervorgerufen. Antinflammatorisch wirkende Mittel können daher auch dazu beitragen die Folgen eines Insektenstiches, wie z.B. Rötung, Quaddelbildung, Juckreiz und schmerzhafte Schwellungen der Haut, abklingen zu lassen, über einen längeren Zeitraum zu unterdrücken und das wieder Auftreten zu verhindern.

Erytheme treten auch verstärkt im Windelbereich von Kleinkindern, um so mehr von Säuglingen auf (Windel Dematitis). Auch Inkontinenz, ein besonders im höheren Alter verstärkt auftretendes Leiden, ist häufig mit Erythemen und Hautrötung als Folge dauernder Exposition mit Feuchtigkeit und Reizstoffen verbunden (Inkontinenz Dermatitis).

Zwar werden in den angesprochenen technischen Bereichen bereits eine Vielzahl Wirkstoffe mit hautirritationsverringernder Wirkung eingesetzt, doch wird weiterhin nach Alternativen gesucht.

In der kosmetischen und pharmazeutischen Industrie besteht daher ein beständiger Bedarf an Mitteln mit hautirritationsverringernder Wirkung. Bei der Suche nach entsprechenden antiinflammatorisch wirksamen Mitteln ist dabei zu beachten, dass die in kosmetischen und/oder pharmazeutischen Produkten verwendeten Substanzen im weiteren
- toxikologisch unbedenklich,
- gut hautverträglich,
- stabil (insbesondere in üblichen kosmetischen und/oder pharmazeutischen Formulierungen),
- vorzugsweise schwach riechend oder (weitgehend) geruchlos,
- vorzugsweise farblos und nicht verfärbend, und
- preiswert herstellbar (d.h. unter Einsatz von Standardverfahren und/oder ausgehend von Standardpräkursoren)
sein müssen.

Die Suche nach geeigneten (Wirk-)substanzen, die eine oder mehrere der genannten Eigenschaften in ausreichendem Maße besitzen, ist dem Fachmann dadurch erschwert, dass keine klare Abhängigkeit zwischen der chemischen Struktur einer Substanz einerseits und ihrer antiinflammatorischen Aktivität sowie ihrer Stabilität andererseits besteht. Des Weiteren gibt es keinen vorhersehbaren Zusammenhang zwischen der antiinflammatorischen Wirkung, der toxikologischen Unbedenklichkeit, der Hautverträglichkeit und/oder der Stabilität.

Es war daher die Aufgabe der vorliegenden Erfindung, einen antiinflammatorischen Wirkstoff anzugeben, der vorzugsweise eine oder mehrere der vorab genannten Nebenbedingungen und besonders bevorzugt alle der vorab genannten Bedingungen erfüllt. Es hat sich überraschenderweise herausgestellt, daß die die kosmetische Verwendung von Alkylamidothiazolen in kosmetischen Zubereitungen zur Prophylaxe vor und Behandlung von sensibler Haut, Juckreiz, trockener Haut, sowie von entzündlichen Erscheinungen in der menschlichen Haut, dadurch gekennzeichnet, daß das oder die Alkylamidothiazole folgende Struktur aufweisen: 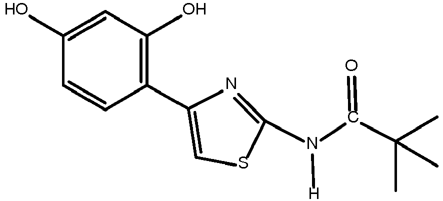 N-(4-(2,4-dihydroxyphenyl)thiazol-2-yl)pivalamide 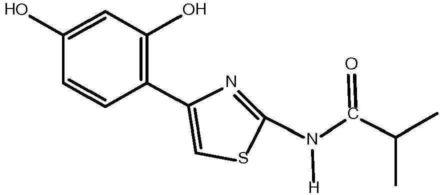 N-(4-(2,4-dihydroxyphenyl)thiazol-2-yl)isobutyramide 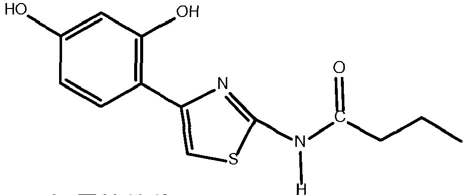 N-(4-(2,4-dihydroxyphenyl)thiazol-2-yl)butyramide 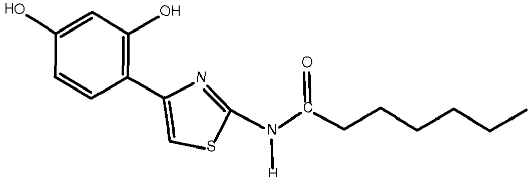 N-(4-(2,4-dihydroxyphenyl)thiazol-2-yl)heptanamide 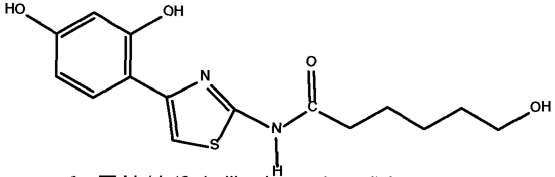 N-(4-(2,4-dihydroxyphenyl)thiazol-2-yl)-6-hydroxyhexanamide 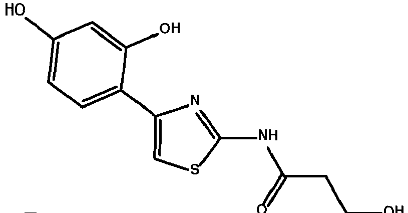 N-(4-(2,4-dihydroxyphenyl)thiazol-2-yl)-3-hydroxypropanamide 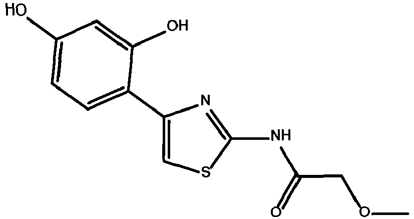 N-(4-(2,4-dihydroxyphenyl)thiazol-2-yl)-2-methoxyacetamide 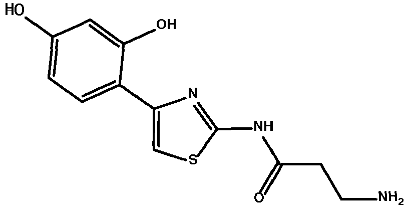 3-amino-N-(4-(2,4-dihydroxyphenyl)thiazol-2-yl)propanamide 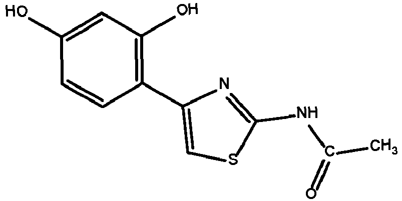 N-(4-(2,4-dihydroxyphenyl)thiazol-2-yl)acetamide 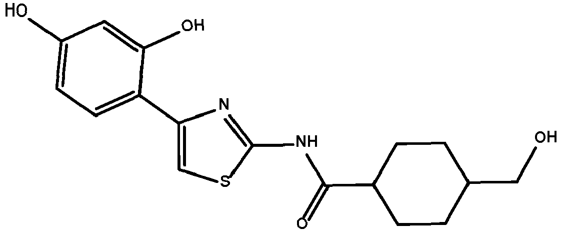 N-(4-(2,4-dihydroxyphenyl)thiazol-2-yl)-4-(hydroxymethyl)cyclohexanecarboxamide 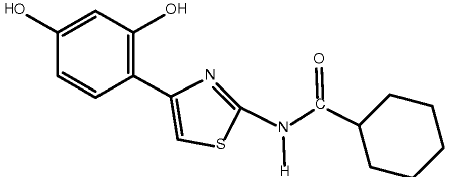 N-(4-(2,4-dihydroxyphenyl)thiazol-2-yl)cyclohexanecarboxamide und 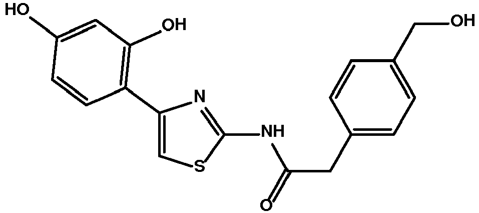 N-(4-(2,4-dihydroxyphenyl)thiazol-2-yl)-2-(4-hydroxymethyl)phenyl)acetamide den Nachteilen das Standes der Technik abhilft.

Erfindungsgemäß sind auch Alkylamidothiazole der vorstehenden Strukturen zur medizinischen Prophylaxe und/oder Behandlung von sensibler Haut, Juckreiz, trockener Haut, sowie von entzündlichen Erscheinungen in der menschlichen Haut.

Bevorzugt enthalten kosmetische oder dermatologische Zubereitungen gemäß der Erfindung 0,000001 bis 10 Gew.-%, besonders bevorzugt 0,0001 bis 3 Gew.-%, und ganz besondres bevorzugt 0,001 bis 1 Gew.-%, an Alkylamidothiazolen, bezogen auf das Gesamtgewicht der Zubereitungen.

Bei Anwendung der erfindungsgemäß verwendeten Wirkstoffe bzw. kosmetischer oder topischer dermatologischer Zubereitungen mit einem wirksamen Gehalt an erfindungsgemäß verwendeter Wirkstoffe ist in überraschender Weise eine wirksame Behandlung von, aber auch eine Prophylaxe bei defizitären, sensitiven oder hypoaktiven Hautzuständen oder defizitären, sensitiven oder hypoaktiven Zustände von Hautanhangsgebilde und/oder entzündlichen Erscheinungen und/oder Juckreiz möglich.

Es ist erfindungsgemäß insbesondere äußerst vorteilhaft, den erfindungsgemäß verwendeten Wirkstoff bzw. kosmetische oder topische dermatologische Zubereitungen mit einem wirksamen Gehalt an erfindungsgemäß verwendetem Wirkstoff zur kosmetischen oder dermatologischen Behandlung oder Prophylaxe unerwünschter Hautzustände zu verwenden.

Die genannten Thiazole können sowohl als freie Base wie auch als Salz vorliegen: z.B. als Fluorid, Chlorid, Bromid, lodid, Sulfat, Carbonat, Ascorbat, Acetat oder Phoshat. Im Besonderen als Halogensalze, wie z.B. Chlorid und Bromid.

Die Prophylaxe bzw. die kosmetische oder dermatologische Behandlung mit dem erfindungsgemäß verwendeten Wirkstoff bzw. mit den kosmetischen oder topischen dermatologischen Zubereitungen mit einem wirksamen Gehalt an erfindungsgemäß verwendetem Wirkstoff erfolgt in der üblichen Weise, und zwar dergestalt, daß der erfindungsgemäß verwendete Wirkstoff bzw. die kosmetischen oder topischen dermatologischen Zubereitungen mit einem wirksamen Gehalt an erfindungsgemäß verwendetem Wirkstoff auf die betroffenen Hautstellen aufgetragen wird.

Vorteilhaft kann der erfindungsgemäß verwendete Wirkstoff eingearbeitet werden in übliche kosmetische und dermatologische Zubereitungen, welche in verschiedenen Formen vorliegen können. So können sie z. B. eine Lösung, eine Emulsion vom Typ Wasser-in-Öl (W/O) oder vom Typ Öl-in-Wasser (O/W), oder eine multiple Emulsionen, beispielsweise vom Typ Wasser-in-ÖI-in-Wasser (W/O/W) oder Öl-in-Wasser-in-Öl (O/W/O), eine Hydrodispersion oder Lipodispersion, eine Pickering-Emulsion, ein Gel, einen festen Stift oder auch ein Aerosol darstellen.

Erfindungsgemäße Emulsionen im Sinne der vorliegenden Erfindung, z. B. in Form einer Creme, einer Lotion, einer kosmetischen Milch sind vorteilhaft und enthalten z. B. Fette, Öle, Wachse und/oder andere Fettkörper, sowie Wasser und einen oder mehrere Emulgatoren, wie sie üblicherweise für einen solchen Typ der Formulierung verwendet werden.

Es ist auch möglich und vorteilhaft im Sinne der vorliegenden Erfindung, den erfindungsgemäß verwendeten Wirkstoff in wäßrige Systeme bzw. Tensidzubereitungen zur Reinigung der Haut und der Haare einzufügen.

Es ist dem Fachmann natürlich bekannt, daß anspruchsvolle kosmetische Zusammensetzungen zumeist nicht ohne die üblichen Hilfs- und Zusatzstoffe denkbar sind. Darunter zählen beispielsweise Konsistenzgeber, Füllstoffe, Parfum, Farbstoffe, Emulgatoren, zusätzliche Wirkstoffe wie Vitamine oder Proteine, Lichtschutzmittel, Stabilisatoren, Insektenrepellentien, Alkohol, Wasser, Salze, antimikrobiell, proteolytisch oder keratolytisch wirksame Substanzen usw.

Mutatis mutandis gelten entsprechende Anforderungen an die Formulierung medizinischer Zubereitungen.

Medizinische topische Zusammensetzungen im Sinne der vorliegenden Erfindung enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z. B. Kosmetikverordnung, Lebensmittel- und Arzneimittelgesetz).

Es ist dabei ebenfalls von Vorteil, den erfindungsgemäß verwendeten Wirkstoff als Zusatzstoff zu Zubereitungen zu geben, die bereits andere Wirkstoffe für andere Zwecke enthalten.

Entsprechend können kosmetische oder topische dermatologische Zusammensetzungen im Sinne der vorliegenden Erfindung, je nach ihrem Aufbau, beispielsweise verwendet werden als Hautschutzcreme, Reinigungsmilch, Sonnenschutzlotion, Nährcreme, Tages- oder Nachtcreme usw. Es ist gegebenenfalls möglich und vorteilhaft, die erfindungsgemäßen Zusammensetzungen als Grundlage für pharmazeutische Formulierungen zu verwenden.

Zur Anwendung werden die kosmetischen und dermatologischen Zubereitungen erfindungsgemäß in der für Kosmetika üblichen Weise auf die Haut und/oder die Haare in ausreichender Menge aufgebracht.

Besonders bevorzugt sind solche kosmetischen und dermatologischen Zubereitungen, die in der Form eines Sonnenschutzmittels vorliegen. Vorteilhaft können diese zusätzlich mindestens einen weiteren UVA-Filter und/oder mindestens einen weiteren UVB-Filter und/oder mindestens ein anorganisches Pigment, bevorzugt ein anorganisches Mikropigment, enthalten.

Die kosmetischen und dermatologischen Zubereitungen können erfindungsgemäß kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, anfeuchtende und/oder feuchhaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Ein zusätzlicher Gehalt an üblichen Antioxidantien ist im allgemeinen bevorzugt. Erfindungsgemäß können als günstige Antioxidantien alle für kosmetische und/oder dermatologische Anwendungen geeigneten oder gebräuchlichen Antioxidantien verwendet werden.

Die Menge der vorgenannten Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern die kosmetische oder dermatologische Zubereitung im Sinne der vorliegenden Erfindung eine Lösung oder Emulsion oder Dispersion darstellt, können als Lösungsmittel verwendet werden:
- Wasser oder wäßrige Lösungen
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder-monobutylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Sofern die kosmetische oder dermatologische Zubereitung im Sinne der vorliegenden Erfindung eine Lösung oder Emulsion oder Dispersion darstellt, können als Lösungsmittel verwendet werden:
- Wasser oder wäßrige Lösungen
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Als Treibmittel für aus Aerosolbehältern versprühbare kosmetische und/oder dermatologische Zubereitungen im Sinne der vorliegenden Erfindung sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die Verwirklichung der vorliegenden Erfindung in Form von Aerosolpräparaten geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Kosmetische Zubereitungen im Sinne der vorliegenden Erfindung können auch als Gele vorliegen, die neben einem wirksamen Gehalt am erfindungsgemäßen Wirkstoff und dafür üblicherweise verwendeten Lösungsmitteln, bevorzugt Wasser, noch organische Verdickungsmittel, z.B. Gummiarabikum, Xanthangummi, Natriumalginat, CelluloseDerivate, vorzugsweise Methylcellulose, Hydroxymethylcellulose, Hydroxyethylcellulose, Hydroxypropylcellulose, Hydroxypropylmethylcellulose oder anorganische Verdickungsmittel, z. B. Aluminiumsilikate wie beispielsweise Bentonite, oder ein Gemisch aus Polyethylenglykol und Polyethylenglykolstearat oder -distearat, enthalten. Das Verdickungsmittel ist in dem Gel z.B. in einer Menge zwischen 0,1 und 30 Gew.-%, bevorzugt zwischen 0,5 und 15 Gew.-%, enthalten.

Es ist erfindungsgemäß vorteilhaft, außer den erfindungsgemäßen Kombinationen weitere öllösliche UVA-Filter und/oder UVB-Filter in der Lipidphase und/oder weitere wasserlösliche UVA-Filter und/oder UVB-Filter in der wäßrigen Phase einzusetzen.

Vorteilhaft können die Lichtschutzformulierungen erfindungsgemäß weitere Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitungen, um kosmetische Zubereitungen zur Verfügung zu stellen, die die Haut vor dem gesamten Bereich der ultravioletten Strahlung schützen. Sie können auch als Sonnenschutzmittel dienen.

### Wirkungsnachweis:

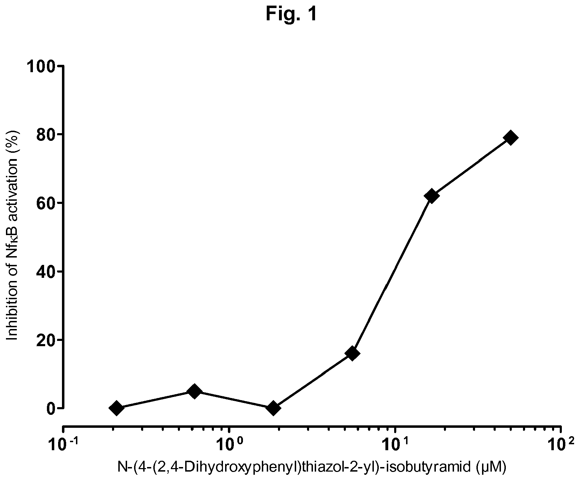

Der nukleäre Faktor kappa B (NFκB) ist ein Master-Regulator der Entzündung. Die Expression vieler pro-inflammatorische Mediatoren (z.B. Zytokine und Chemokine) steht unter der Kontrolle von NFκB. Die Inhibition der Aktivierung von NFκB ist deshalb ein wichtiger Mechanismus um Entzündungsvorgänge zu minimieren. Zur Messung der NFκB Aktivität wurde ein kommerzieller Assay der Firma Cell Culture Service GmbH, Hamburg, eingesetzt. Bei diesem Test wird NFκB durch Zugabe des pro-entzündlichen Mediators TNF-alpha aktiviert. Die Zugabe der erfindungsgemäßen Alkylamidothiazole verhindert dosis-abhängig den TNF-alpha Effekt. In Fig. 1 ist exemplarisch der gezeigt, dass schon sehr geringe Konzentrationen von N-(4-(2,4-Dihydroxyphenyl)thiazol-2-yl)-isobutyramid die TNF-alpha induzierte Aktivierung von NFκB verhindern.

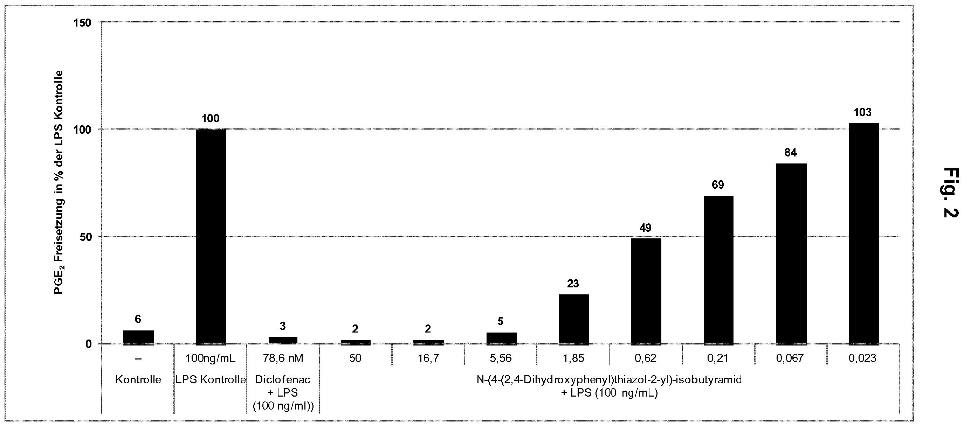

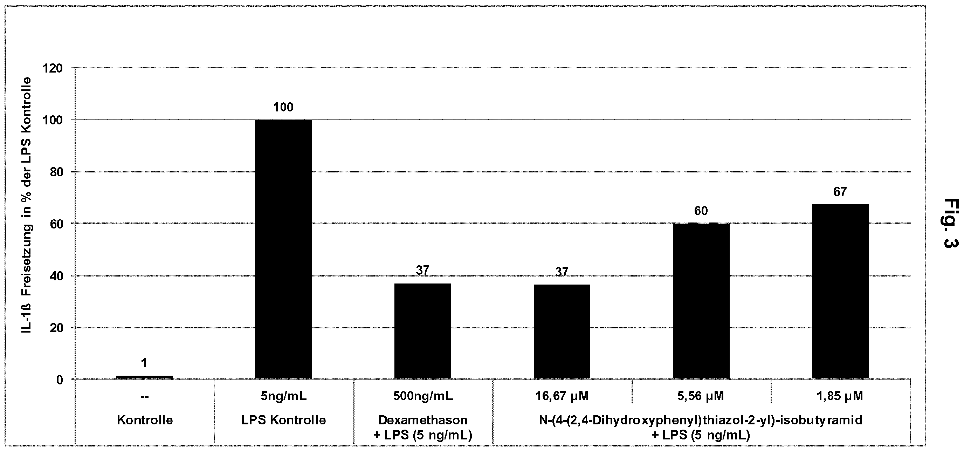

In weiteren Experimenten wurde untersucht, ob die inhibiton der NFκB Aktivierung auch tatsächlich die nachfolgende Ausschüttung pro-inflammatorischer Mediatoren verringert. Exemplarisch sind dazu die Ergebnisse von zwei Experimenten in den Abbildungen dokumentiert. Durch Inkubation humaner dermaler Fibroblasten mit Lipopolysaccharide (LPS) wurde in diesen Zellen die Bildung des pro-inflammatorischen Prostaglandin E₂ induziert und mittels eines spezifischen ELISA die Konzentration im Zellkulturüberstand, nach 24 Stunden Inkubation mit LPS und ansteigenden Konzentrationen der erfindungsgemäßen Substanzen, bestimmt. Wie aus Fig. 2 ersichtlich reduziert N-(4-(2,4-Dihydroxyphenyl)thiazol-2-yl)-isobutyramid sehr signifikant die Freisetzung von PGE₂ auf ein Niveau, das vergleichbar ist zum des pharmakologischen Standards Diclofenac. Im nächsten Experiment wurde die Freisetzung Interleukin 1β (IL-1β) aus Blutleukozyten nach Stimulierung durch LPS und gleichzeitiger Inkubation (für 24 Stunden) mit den erfindungsgemäßen Substanzen gemessen. Auch hier zeigen Alkylamidothiazole (hier exemplarisch N-(4-(2,4-Dihydroxyphenyl)thiazol-2-yl)-isobutyramid) eine sehr gute antientzündliche Wirksamkeit. Bei einer Konzentration von 16,67 µM wird eine Wirksamkeit wie bei 500 ng Dexamethason, einem super-potenten Corticosteroid, erreicht. Das Antientzündliche Wirkniveau der Alkylamidothiazole liegt also bei dem anerkannter pharmakologischer Standards, auch wenn dafür höhere Konzentrationen der erfindungsgemäßen Substanzen eingesetzt werden müssen.

### Svnthesevorschriften exemplarisch ausgewählter Alkylamidothiazole:

### 2-Brom-2',4'-bis-methoxycarbonyloxy-acetophenon:

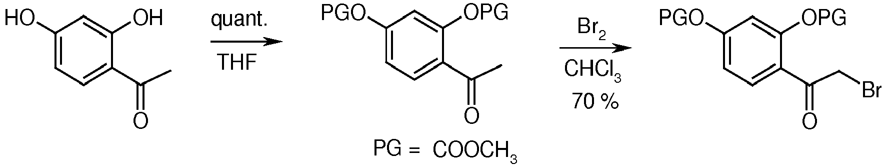

Mitchell, David; Doecke, Christopher W.; Hay, Lynne A.; Koenig, Thomas M.; Wirth, David D. Tetrahedron Letters, 1995

Eine Lösung von 60g (369 mmol) 2,4-Dihydroxyacetophenon and 186 ml Triethylamin in 900 ml Tetrahydrofuran wurde auf 0°C gekühlt und 93 ml Chlorameisensäuremethylester in 400 ml Tetrahydrofuran langsam zugetropft. Es bildet sich ein weißer Niederschlag. Nach 3 Stunden Rühren bei Raumtemperatur ist die Reaktion abgeschlossen (DC-Kontrolle). Der Niederschlag wurde abgesaugt und mit reichlich Tetrahydrofuran gewaschen. Das Filtrat wurde zur Trockne einrotiert, in Ethylacetat aufgenommen mit 1N HCl und NaCI-Lösung (sat.) gewaschen und über Magnesiumsulfat getrocknet, vom Magnesiumsulfat filtriert und das Ethylacetat am Rotationsverdampfer eingeengt. Es wurden 105 g von 2,4-Bis-methoxycarbonyloxy-acetophenon erhalten.¹H NMR (DMSO-D₆): 8.05 (d, 1H), 7.38 (d, 1H), 7.36 (s, 1H), 3.86 (d, 6H). Das Produkt wurde ohne weitere Reinigung eingesetzt. Zu der Lösung von 105 g 2,4-Bis-methoxycarbonyloxy-acetophenon in Chloroform (1000 ml) wurden 63g (392 mmol) Brom in 450 ml Chloroform innerhalb von 3 h zugetropft. Danach wurde die Reaktion noch 15 min. bei Raumtemperatur gerührt, Das Lösungsmittel wurde einrotiert. Der Rückstand wurde in Ethylacetat/n-Hexan verrührt, der entstandene Niederschlag wurde abgesaugt. Umkristallisation aus Ethylacetat/n-Hexan lieferten 100 g 2-Brom-2',4'- bis-methoxycarbonyloxy-acetophenon. ¹H NMR (DMSO-D₆): 8.11 (d, 1H), 7.42 (m, 2H), 4. 87 (s, 2H), 3.87 (s, 3H), 3.85 (s, 3H) ppm; m.p. 73-74°C.

### N-(4-(2,4-Dihydroxyphenyl)thiazol-2-yl)-pivalamid:

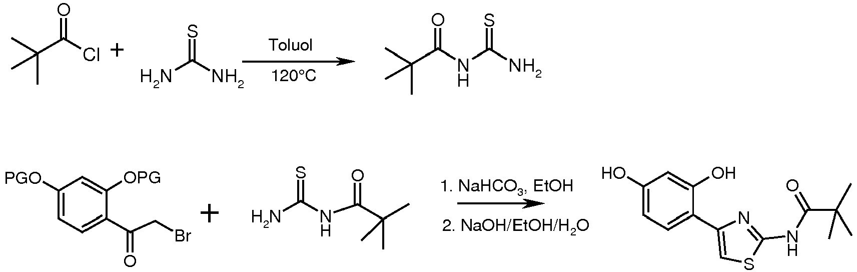

126 g (1.66 mmol) Thioharnstoff wurden in Toluol (1000 ml) vorlegt und 100 g (829 mmol) Pivaloylchlorid zugetropft. Die Reaktionslösung wurde 3 Stunden unter Rückfluss gekocht, wobei 2 Phasen entstehen. Die obere Phase wurde abdekantiert und abgekühlt. Die ausgefallenen farblosen Nadeln wurden abgesaugt und mit Cyclohexan gewaschen und im Vakuum getrocknet. Ausbeute: 64 g. ¹H NMR (DMSO-D6): 10.27 (s, 1H), 9.74 (s, 1H), 9.40 (s, 1H), 1.19 (s, 9H) ppm.

107.7 g (310 mmol) 2-Brom-2',4'- bis-methoxycarbonyloxy-acetophenon wurden mit 49.7 g (13.6 mmol) N-Pivaloylthioharnstoff und 39.2 g (466 mmol) NaHCO₃ in 1.2l Ethanol unter Rückfluss für 0.5h gekocht. Die Reaktionslösung wurde abgekühlt und mit 50.6 g (1.27 mol) NaOH in 250 ml Wasser versetzt. Nach 30 Min. Rühren bei Raumtemperatur wurde die Reaktionslösung mit 300 ml Wasser aufgenommen und mit 2N HCl neutralisiert. Der entstandene Niederschlag wurde abfiltriert und aus Ethanol/Wasser umkristallisiert. Es wurden 80 g Thiazol erhalten. ¹H NMR (DMSO-D₆): 11.77 (bs, 1H), 11.02 (bs, 1H), 9.47 (bs, 2H), 7.65 (d, 1H), 7.39 (s, 1H), 6.30 (s, 1H), 6.28 (d, 1H), 1.27 (s, 9H) ppm; m.p. 257-259°C.

### N-(4-(2,4-Dihydroxyphenyl)thiazol-2-yl)-isobutyramid:

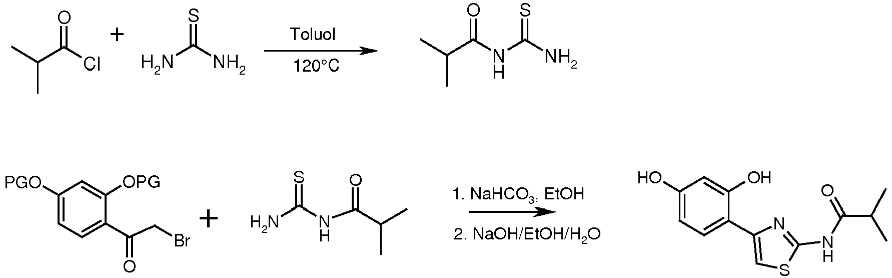

114 g (1.5 mol) Thioharnstoff wurden in Toluol (800 ml) vorgelegt und 80 g (0.75 mol) Isobutyrylchlorid zugetropft. Die Reaktionslösung wurde 3 Stunden unter Rückfluss gekocht, wobei 2 Phasen entstehen. Die obere Phase wurde abdekantiert und abgekühlt. Die ausgefallenen weißen Kristalle wurden abgesaugt und mit Toluol gewaschen und im Vakuum getrocknet. Ausbeute: 62 g. ¹H NMR (DMSO-D₆): 11.03 (bs, 1H), 9.66 (bs, 1H), 9.35 (bs, 1H), 2.72 (m, 1H), 1.03 (2, 6H) ppm;
89 g (260 mmol) 2-Brom-2',4'- bis-methoxycarbonyloxy-acetophenon wurden mit 37.5 g (260 mmol) N-Isobutyrylthioharnstoff und 32 g (380 mmol) NaHCO₃ in 1000 ml Ethanol unter Rückfluss für 0.5h gekocht. Die Reaktionslösung wurde abgekühlt und mit 41 g (0.93 mol) NaOH in 250 ml Wasser versetzt. Nach 30 Min. Rühren bei Raumtemperatur wurde die Reaktionslösung mit 300 ml Wasser aufgenommen und mit 2N HCl auf pH=3 eingestellt. Der entstandene Niederschlag wurde abfiltriert und aus Ethanol/Wasser umkristallisiert. Es wurden 56 g Thiazol erhalten. ¹H NMR (DMSO-D₆): 12.16 (bs, 1H), 10.88 (bs, 1H), 9.47 (bs, 1H), 7.65 (m, 1H), 7.41 (s, 1H), 6.32 (m, 2H), 2.75 (m, 1H), 1.14 (d, 6H) ppm. m.p.: 243-245°C.

### N-(4-(2,4-Dihydroxyphenyl)thiazol-2-yl)-butyramid:

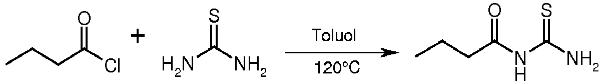

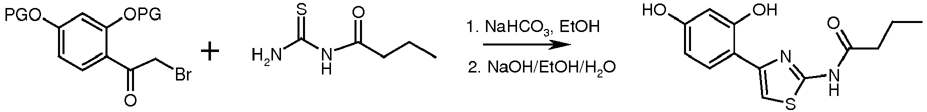

143 g (1.88 mol) Thioharnstoff wurden in Toluol (1000 ml) vorgelegt und 100 g (0.93 mol) n-Butyrylchlorid zugetropft. Die Reaktionslösung wurde 3 Stunden unter Rückfluss gekocht, wobei 2 Phasen entstehen. Die obere Phase wurde abdekantiert und abgekühlt. Die ausgefallenen leicht gelblichen Kristalle wurden abgesaugt und mit Toluol gewaschen und im Vakuum getrocknet. Ausbeute: 88 g. ¹H NMR (DMSO-D₆): 11.03 (bs, 1H), 9.65 (bs, 1H), 9.33 (bs, 1H), 2.33 (t, 2H), 1.53 (m, 2H), 0.86 (t, 3H) ppm; m.p.: 115-188°C

92 g (265 mmol) 2-Brom-2',4'- bis-methoxycarbonyloxy-acetophenon wurden mit 38.75 g (265 mmol) N-Butyrylthioharnstoff und 34 g (397 mmol) NaHCO₃ in 900 ml Ethanol unter Rückfluss für 0.5h gekocht. Die Reaktionslösung wurde abgekühlt und mit 37 g (0.93 mol) NaOH in 300 ml Wasser versetzt. Nach 30 Min. Rühren bei Raumtemperatur wurde die Reaktionslösung mit 300 ml Wasser aufgenommen und mit 2N HCl neutralisiert. Der entstandene Niederschlag wurde abfiltriert und aus Ethanol/Wasser umkristallisiert. Es wurden 67 g Thiazol erhalten. ¹H NMR (DMSO-D₆): 12.18 (bs, 1H), 10.89 (bs, 1H), 9.48 (bs, 1H), 7.65 (1 arom. H), 7.40 (s, 1H), 6.31 (2 arom. H), 2.43 (t, 2H), 1.64 (m, 2H), 0.91 (t, 3H)ppm. m.p.: 227-229°C.

### N-(4-(2,4-Dihydroxyphenyl)thiazol-2-yl)-acetamid:

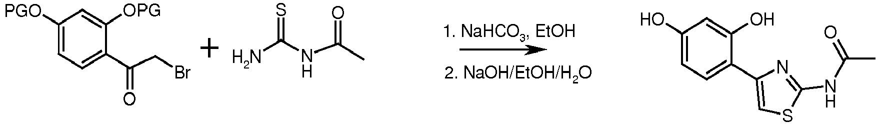

4.71 g (13.6 mmol) von 2-Brom-2',4'- bis-methoxycarbonyloxy-acetophenon wurden mit 1.61 g (13.6 mmol) N-Acetylthioharnstoff und 1.72 g (20.4 mmol) NaHCO₃ in 45 ml Ethanol 0.5h unter Rückfluss gekocht. Die Reaktionslösung wurde abgekühlt und mit 2.0 g (50 mmol) NaOH in 20 ml Wasser versetzt. Nach 20 Min. Rühren bei 0°C wurde die Reaktionslösung mit 30 ml Wasser aufgenommen und mit halbkonz. HCl neutralisiert. Der entstandene Niederschlag wurde abfiltriert und aus Ethanol/Wasser umkristallisiert. Es wurden 2.73g Produkt erhalten. ¹H NMR (DMSO-D₆): 12.20 (b, 1H), 10.85 (s, 1H), 9.46 (s, 1H), 7.64 (m, 1H), 7.38 (s, 1H), 6.28 (m, 2H), 2.15 (s, 3H) ppm; m.p. 264-264°C.

### N-(4-(2,4-Dihydroxyphenyl)thiazol-2-yl)-4-(Hydroxymethyl)cyclohexancarboxamid:

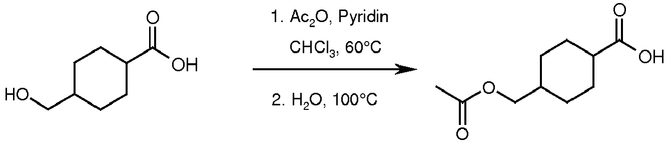

Durchführung analog Literatur. BANYU Pharmaceutical Co., Ltd., EP2072519 A1, 2009 Ausbeute: 96%, ¹H NMR (DMSO-D₆): 12.03 (bs, 1H), 3.85, 3.82 (2 x d, 2H), 2.50, 2.47 (2 x m, 1H), 2.00 (s, 3H), 0.95-1.90 (m, 9H) ppm;

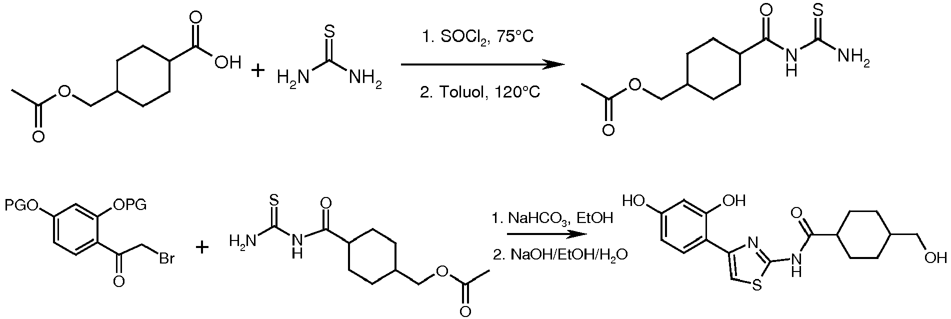

95 g (0.47 mol) 4-Acetoxymethylcyclohexancarbonsäure wurden in 350 ml Thionylchlorid 2h unter Rückfluss erhitzt. Nach Entfernen des überschüssigen Thionylchlorids im Vakuum wurde der Rückstand in 1l Toluol aufgenommen und 71 g (0.94 mol) Thioharnstoff zugegeben. Die Reaktionslösung wurde 3 Stunden unter Rückfluss gekocht und anschließend heiß filtriert. Nach Abkühlen der Mutterlauge wurden die entstandenen weißen Kristalle abgesaugt, mit Toluol gewaschen und im Vakuum getrocknet. Ausbeute: 59 g. ¹H NMR (DMSO-D₆): 11.03, 10.97 (2 x s, 1H), 9.64 (bs, 1H), 9.35 (bs, 1H), 3.93, 3.82 (2 x d, 2H), 2.61, 2.42 (2 x m, 1H), 2.00 (s, 3H), 1.60 (m, 8H), 1.35, 0.94 (2 x m, 1H) ppm;
79 g (228 mmol) 2-Brom-2',4'- bis-methoxycarbonyloxy-acetophenon wurden mit 59 g (228 mmol) N-(4-Acetoxymethylcyclohexylcarbonyl)thioharnstoff und 29 g (340 mmol) NaHCO₃ in 1000 ml Ethanol unter Rückfluss für 0.5h gekocht. Die Reaktionslösung wurde abgekühlt und mit 73 g (1.8 mol) NaOH in 300 ml Wasser versetzt. Nach 30 Min. Rühren bei Raumtemperatur wurde die Reaktionslösung mit 300 ml Wasser aufgenommen und mit 2N HCl auf pH=3 eingestellt. Der entstandene Niederschlag wurde abfiltriert und aus Ethanol/Wasser umkristallisiert. Es wurden 47 g Thiazol erhalten. ¹H NMR (DMSO-D₆): 12.15, 12.10 (2 x s, 1H), 10.96 (2 x s, 1H), 9.47 (br, 2H), 7.64 (d, 1H), 7.39 (s, 1H), 6.29 (m, 2H), 4.40 (br, 1H), 3.32, 3.23 (2 x d, 2H), 2.65, 2.44 (2 x m, 1H), 1.90 (m, 1H), 1.78 (m, 2H), 1.50 (m, 5H), 0.94 (m, 1H) ppm. m.p.: 152-160°C.

### N-(4-(2,4-Dihydroxyphenyl)thiazol-2-yl)-cyclohexancarboxamid:

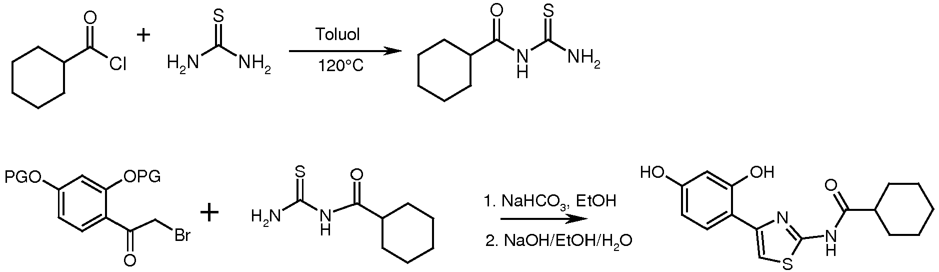

52 g (0.68 mol) Thioharnstoff wurden in Toluol (500 ml) vorgelegt und 50 g (0.34 mol) Cyclohexanoylchlorid zugetropft. Die Reaktionslösung wurde 3 Stunden unter Rückfluss gekocht, wobei 2 Phasen entstehen. Die obere Phase wurde abdekantiert und abgekühlt. Die ausgefallenen Kristalle wurden abgesaugt, mit Toluol gewaschen und aus Methanol umkristallisiert. Ausbeute: 35 g. ¹H NMR (DMSO-D₆): 10.98 (bs, 1H), 9.65 (bs, 1H), 9.32 (bs, 1H), 2.49 (t, 1H), 1.75 (m, 4H), 1.61 (m, 1H), 1.18 (m, 5H) ppm.

92 g (265 mmol) 2-Brom-2',4'- bis-methoxycarbonyloxy-acetophenon wurden mit 49.4 g (265 mmol) N-Cyclohexanoylthioharnstoff und 34 g (397 mmol) NaHCO₃ in 900 ml Ethanol unter Rückfluss für 0.5h gekocht. Die Reaktionslösung wurde abgekühlt und mit 37 g (930 mmol) NaOH in 300 ml Wasser versetzt. Nach 30 Min. Rühren bei Raumtemperatur wurde die Reaktionslösung mit 300 ml Wasser aufgenommen und mit 2N HCl neutralisiert. Das Ethanol wurde weitgehend am Rotationsverdampfer entfernt. Der entstandene Niederschlag wurde abfiltriert und aus Ethanol/Wasser umkristallisiert. Es wurden 70 g Thiazol erhalten. ¹H NMR (DMSO-D₆): 12.14 (bs, 1H), 11.00 (bs, 1H), 9.48 (bs, 1H), 7.64 (1 arom. H), 7.39 (s, 1H), 6.30 (2 arom. H), 2.49 (m, 1H), 1.84 (m, 2H), 1.76 (m, 2H), 1.65 (m, 1H), 1.42 (m, 2H), 1.25 (m, 3H) ppm. m.p.: 262-266°C.

### N-(4-(2,4-dihydroxyphenyl)thiazol-2-yl)-2-(4-(hydroxymethyl)phenyl)acetamid:

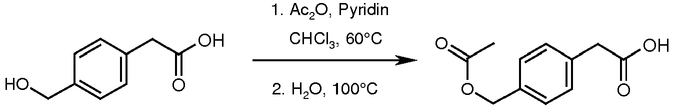

Durchführung analog Literatur. BANYU Pharmaceutical Co., Ltd., EP2072519 A1, 2009 Ausbeute: 76%, ¹H NMR (DMSO-D₆): 12.31 (bs, 1H), 7.26 (m, 4H), 5.05 (s, 2H), 3.57 (s, 2H), 2.05 (s, 3H) ppm;

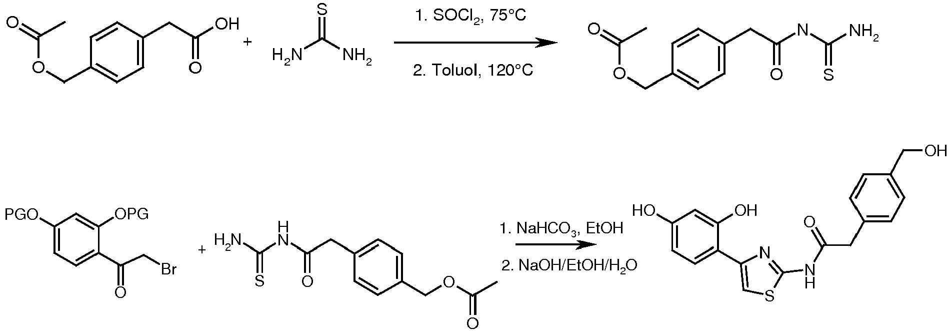

3.7 g (18 mmol) 4-Acetoxymethylphenylessigsäure wurden in 40 ml Thionylchlorid 2 h unter Rückfluss erhitzt. Nach Entfernen des überschüssigen Thionylchlorids im Vakuum wurde der Rückstand in 70 ml Toluol aufgenommen und 2.7 g (36 mmol) Thioharnstoff zugegeben. Die Reaktionslösung wurde 3 Stunden unter Rückfluss gekocht und anschließend wurde das Lösungsmittel im Vakuum entfernt. Die Reinigung erfolgte mittels Säulenchromatographie mit Cyclohexan/Essigester 1/1 an Kieselgel. Ausbeute: 2.7 g. ¹H NMR (DMSO-D₆): 11.29 (bs, 1H), 9.55 (bs, 1H), 9.40 (bs, 1H), 7.30 (m, 4H), 5.04 (s, 2H), 3.71 (s, 2H), 2.05 (s, 3H) ppm;

3.5 g (10 mmol) 2-Brom-2',4'- bis-methoxycarbonyloxy-acetophenon wurden mit 2.7 g (10 mmol) N-[2-(4-Acetoxymethylphenyl)acetyl]thioharnstoff und 1.3 g (15 mmol) Na-HCO₃ in 50 ml Ethanol unter Rückfluss für 0.5h gekocht. Die Reaktionslösung wurde abgekühlt und mit 4.0 g (0.1 mol) NaOH in 20 ml Wasser versetzt. Nach 2 h Rühren bei 60°C wurde die Reaktionslösung in 100 ml Wasser aufgenommen und mit 2N HCl auf pH=3 eingestellt. Der entstandene Niederschlag wurde abfiltriert und aus Ethanol/Wasser umkristallisiert. Es wurden 1.3 g Thiazol erhalten. ¹H NMR (DMSO-D₆): 12.44 (s, 1H), 10.80 (s, 1H), 9.48 (s, 1H), 7.66 (d, 1H), 7.41 (s, 1H), 7.29 (m, 4H), 6.32 (m, 2H), 5.13 (t, 1H), 4.47 (d, 2H), 3.77 (s, 2H) ppm. m.p.: 254-256°C.

Die nachfolgenden Beispiele sollen die vorliegende Erfin-dung verdeutlichen, ohne sie ein-zu-schränken. Alle Mengen-angaben, Anteile und Prozentanteile sind, soweit nicht an-ders an-gegeben, auf das Gewicht und die Gesamtmenge bzw. auf das Ge-samt-gewicht der Zu-be-reitungen bezogen.

### Rezepturbeispiele

### O/W -Emulsionen

| **Rezepturbeispiele** | **1** | **2** |
|---|---|---|
| **Chemische Bezeichnung** | **Gew.-%** | **Gew.-%** |
| PEG-40 Stearate | 0,80 | 1,00 |
| Glyceryl Stearate | 2,50 | 3,00 |
| C12-15 Alkyl Benzoate | 2,00 | 2,50 |
| Caprylsäure/Caprinsäure Triglycerid | 2,00 | 2,50 |
| Cetylstearylalkohol | 3,00 | 3,00 |
| Cyclomethicon | 2,00 | 2,00 |
| Dicaprylyl Carbonat | - | 2,00 |
| Octyldodecanol | 1,00 | - |
| Triisostearin | - | 0,50 |
| Butyrospermum Parkii Butter | 2,00 | - |
| **Octyldodecyl Myristat** | 1,00 | - |
| Dimethicon | 1,00 | 1,00 |
| Glycerin | 7,50 | 5,00 |
| Methylparaben | 0,20 | - |
| Phenoxyethanol | 0,40 | 0,50 |
| Propylparaben | 0,10 | - |
| Glyceryl Caprylat | - | 0,25 |
| Pentylenglykol | - | 0,50 |
| Butylenglykol | 1,00 | - |
| Lauroylethylarginat | 0,10 | 0,05 |
| Kaliumsorbat | 0,10 | 0,05 |
| Natriumsalicylat | 0,01 | 0,02 |
| Carbomer | 0,15 | 0,10 |
| Natrium Polyacrylat | - | 0,20 |
| Xanthan Gummi | 0,10 | - |
| Acrylates/C10-30 Alkyl Acrylate Crosspolymer | - | 0,10 |
| Trinatrium EDTA + Wasser (20%ige wässrige Lösung) | - | 1,00 |
| Tapioca Stärke | - | 1,00 |
| Distärke Phosphat | - | 1,00 |
| Aluminum Stärke Octenylsuccinat | 2,00 | - |
| Acrylonitrile-methacrylonitrile-methylmethacrylate Copolymer + Isopentane + Magnesium Hydroxide | 1,00 | - |
| N-(4-(2,4-Dihydroxyphenyl)thiazol-2-yl)-isobutyramid | 0,10 | 0,15 |
| Ethylhexyl Methoxycinnamat | 1,00 | 2,00 |
| Diethylamino Hydroxybenzoyl Hexyl Benzoat | 0,50 | 1,00 |
| Homosalate (3,3,5-Trimethylcyclohexylsalicylat) | 2,00 | 2,00 |
| Phenylbenzimidazole Sulfonsäure | 1,00 | 1,00 |
| Titandioxid | - | 1,00 |
| Glyceryl Glucoside | 3,00 | - |
| Kurzkettige Hyaluronsäure | - | 0,10 |
| Langkettige Hyaluronsäure | 0,10 | - |
| 4-Butyl-Resorcin | - | 0,10 |
| Magnolia-Rinden-Extrakt | 0,10 | - |
| Octadecen Dioic Säure | - | 0,05 |
| Folsäure | 0,05 | - |
| Carnitin | - | 0,2 |
| Kreatin | 0,10 | - |
| Alpha-Glucosylrutin | - | 0,01 |
| Taurin | 0,20 | - |
| Maulbeeren-Wurzel-Extrakt | - | 0,05 |
| Natrium Metabisulfit | 0,10 | - |
| **Diethylhexyl Syringylidenmalonat** | 0,13 | 0,13 |
| Natriumhydroxid | q.s. | q.s. |
| 3-Methyl-5-phenyl-1-pentanol | 0,10 | - |
| Coumarin | - | 0,05 |
| Ethyllinalool | 0,01 | - |
| Ascorbylpalmitat | 0,10 | - |
| Parfüm | q.s. | q.s. |
| Wasser | ad 100 | ad 100 |

| **Rezepturbeispiel** | **3** | **4** |
|---|---|---|
| **Chemische Bezeichnung** | **Gew.-%** | **Gew.-%** |
| Glyceryl Stearat Citrat | 2,00 | 1,50 |
| Behenyl Alkohol | 1,50 | 1,00 |
| C12-15 Alkyl Benzoat | 2,00 | 2,50 |
| Caprylsäure/Caprinsäure Triglycerid | 2,00 | 2,00 |
| Cetyl Alkohol | 2,00 | 2,00 |
| Cetylstearylalkohol | 2,00 | - |
| Cyclopentasiloxane | - | 1,00 |
| Cyclomethicon | 1,00 | 1,00 |
| Dicaprylyl Carbonat | - | 2,00 |
| Paraffinum Liquidum (Mineralöl) | 1,00 | - |
| Octyldodecanol | - | 2,00 |
| Isopropyl Palmitate | 1,50 | - |
| Dimethicon | 0,50 | 1,00 |
| Glycerin | 3,00 | 5,00 |
| Methylparaben | 0,20 | 0,15 |
| Phenoxyethanol | 0,40 | 0,60 |
| Propylparaben | 0,10 | - |
| Methylisothiazolinon | - | 0,05 |
| Piroctone Olamine | 0,10 | - |
| Natriumbenzoat | 0,10 | 0,10 |
| Glyceryl Caprylat | - | 0,50 |
| Ethylparaben | 0,10 | - |
| Carbomer | 0,20 | - |
| Natrium Polyacrylat | - | 0,40 |
| Xanthan Gummi | 0,10 | - |
| Acrylates/C10-30 Alkyl Acrylate Cross-polymer | - | 0,10 |
| Tapioka Stärke | 0,50 | - |
| Nylon-12 (1,8-Diazacyclotetradecan-2,7-dion Homopolymer) | 1,00 | - |
| Polymethylsilsesquioxane | - | 1,00 |
| Aluminum Stärke Octenylsuccinat | - | 1,00 |
| N-(4-(2,4-Dihydroxyphenyl)thiazol-2-yl)-isobutyramid | 0.25 | 0.15 |
| N-(4-(2,4-Dihydroxyphenyl)thiazol-2-yl)-pivalamid | 0.10 | 0.10 |
| N-(4-(2,4-Dihydroxyphenyl)thiazol-2-yl)-butyramid | 0.01 | 0.25 |
| N-(4-(2,4-Dihydroxyphenyl)thiazol-2-yl)-cyclohexancarboxamid | 0,20 | 0.10 |
| **Glycyrrhiza Inflata Root Extrakt** | 0,03 | 0,05 |
| Titandioxid | - | 1,00 |
| Octocrylene | 1,00 | 2,00 |
| Bis-Ethylhexyloxyphenol Methoxyphenyl Triazin | 1,00 | 1,00 |
| 2-Ethylhexyl Methoxycinnamat | 2,00 | 2,00 |
| Homosalat (3,3,5-Trimethylcyclohexylsalicylat) | 1,00 | 1,00 |
| Natriumhydroxid | q.s. | q.s. |
| Trinatrium EDTA | 0,15 | - |
| 1-(1,2,3,4,5,6,7,8-octahydro-2,3,8,8,-tetramethyl-2-naphthyl)ethan-1-one | 0,1 | - |
| Geraniol | - | 0,05 |
| Hexylcinnamal | 0,10 | - |
| Parfüm | 0,10 | 0,20 |
| Wasser | ad 100 | ad 100 |

| **Rezepturbeispiele** | **5** | **6** |
|---|---|---|
| **Chemische Bezeichnung** | **Gew.-%** | **Gew.-%** |
| Polyglyceryl-3 Methylglucose Distearat | 2,00 | 2,50 |
| Sorbitan Stearate | 1,50 | 3,00 |
| C12-15 Alkyl Benzoat | 2,50 | 2,50 |
| Caprylsäure/Caprinsäure Triglyceride | 2,50 | 2,50 |
| Stearyl Alkohol | 1,00 | 1,50 |
| Cyclomethicon | 3,00 | 1,00 |
| Isopropyl Myristat | - | 2,50 |
| Isopropyl Palmitat | 2,00 | - |
| EThylhexyl Stearat | - | 1,50 |
| Dimethicon | - | 1,00 |
| .Decyl Oleate | - | 1,50 |
| Glycerin | 5,00 | 7,50 |
| Butyrospermum Parkii Butter | 2,00 | - |
| Squalane | 0,50 | - |
| Natriumbenzoat | 0,10 | 0,10 |
| Methylparaben | 0,20 | 0,20 |
| Phenoxyethanol | 0,40 | 0,40 |
| Propylparaben | 0,10 | - |
| Benzethonium chlorid | - | 0,10 |
| Caprylyl Glykol | - | 0,20 |
| Ethylhexylglycerin | - | 0,20 |
| Pentylen Glykol | - | 0,10 |
| Carbomer | 0,15 | 0,10 |
| Ammonium Acryloyldimethyltaurate/VP Copolymer | - | 0,20 |
| Carrageenan | 0,10 | - |
| Trinatrium EDTA | - | 1,00 |
| Tapioka Stärke | - | 1,00 |
| Distärke Phosphat | - | 1,00 |
| Acrylonitrile-methacrylonitrile-methyl-methacrylate Copolymer + Isopentane + Magnesium Hydroxide | 1,00 | - |
| N-(4-(2,4-Dihydroxyphenyl)thiazol-2-yl)-isobutyramid | 0.01 | 0.25 |
| N-(4-(2,4-Dihydroxyphenyl)thiazol-2-yl)-pivalamid | 0,20 | 0.10 |
| N-(4-(2,4-Dihydroxyphenyl)thiazol-2-yl)-butyramid | 0.25 | 0.15 |
| N-(4-(2,4-Dihydroxyphenyl)thiazol-2-yl)-cyclohexancarboxamid | 0.10 | 0.10 |
| Diethylamino Hydroxybenzoyl Hexyl Benzoate | 1,00 | 2,00 |
| Ethylhexyl Methoxycinnamat | 1,00 | 1,00 |
| Butyl Methoxydibenzoylmethan | 2,00 | 2,00 |
| Octocrylen | 1,00 | 1,00 |
| Titandioxid | - | 0,50 |
| Natriumhydroxid | q.s. | q.s. |
| Ubiquinone | 0,10 | - |
| Natrium Metabisulfit | - | 0,15 |
| BHT (tert-Butylhydroxytoluol) | 0,10 | - |
| Linalyacetat | 0,05 | - |
| Hexylsalicylat | - | 0,05 |
| Benzylsalicylat | 0,10 | - |
| Parfüm | q.s. | q.s. |
| Wasser | ad 100 | ad 100 |

## Patentansprüche

1. Kosmetische Verwendung von Alkylamidothiazolen in kosmetischen Zubereitungen zur Prophylaxe vor und Behandlung von trockener Haut in der menschlichen Haut, **dadurch gekennzeichnet, daß** das oder die Alkylamidothiazole folgende Struktur aufweisen: 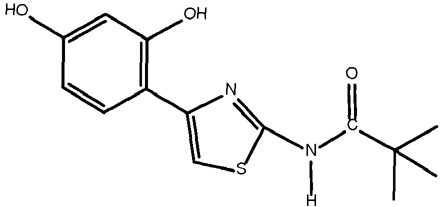 N-(4-(2,4-dihydroxyphenyl)thiazol-2-yl)pivalamide 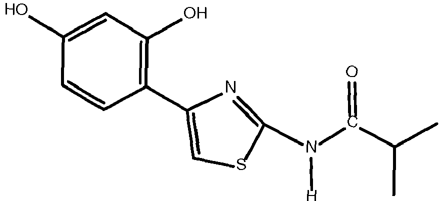 N-(4-(2,4-dihydroxyphenyl)thiazol-2-yl)isobutyramide 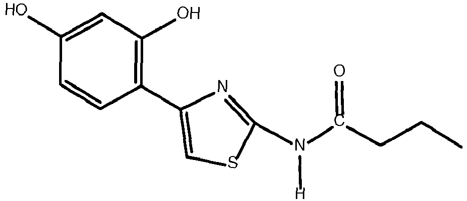 N-(4-(2,4-dihydroxyphenyl)thiazol-2-yl)butyramide 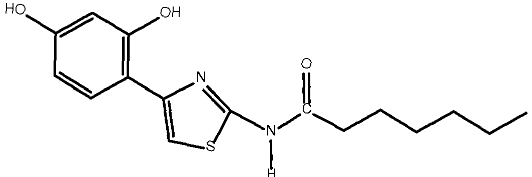 N-(4-(2,4-dihydroxyphenyl)thiazol-2-yl)heptanamide 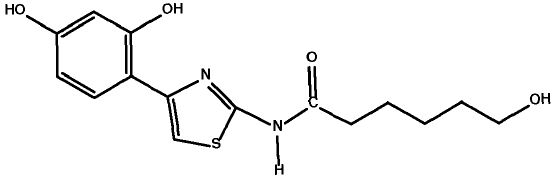 N-(4-(2,4-dihydroxyphenyl)thiazol-2-yl)-6-hydroxyhexanamide 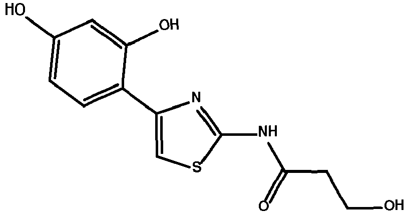 N-(4-(2,4-dihydroxyphenyl)thiazol-2-yl)-3-hydroxypropanamide 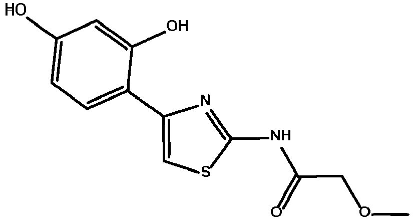 N-(4-(2,4-dihydroxyphenyl)thiazol-2-yl)-2-methoxyacetamide 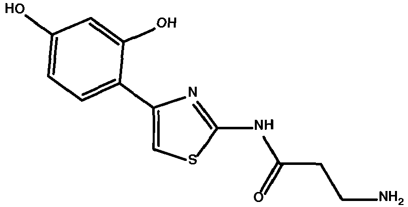 3-amino-N-(4-(2,4-dihydroxyphenyl)thiazol-2-yl)propanamide 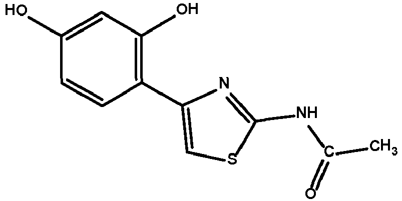 N-(4-(2,4-dihydroxyphenyl)thiazol-2-yl)acetamide 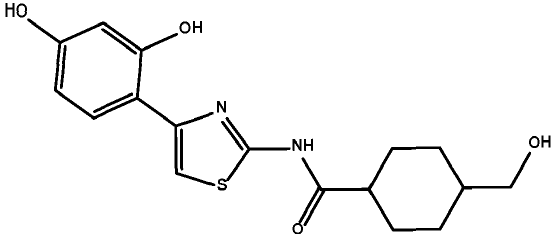 N-(4-(2,4-dihydroxyphenyl)thiazol-2-yl)-4-(hydroxymethyl)cyclohexanecarboxamide 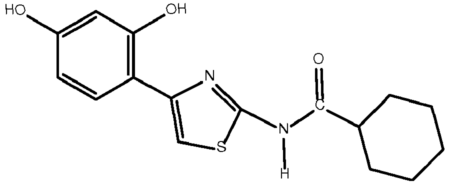 N-(4-(2,4-dihydroxyphenyl)thiazol-2-yl)cyclohexanecarboxamide und 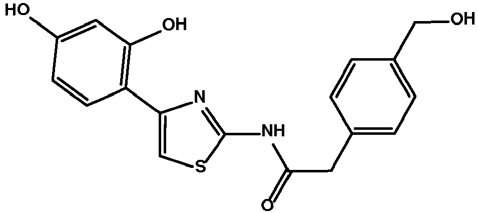 N-(4-(2,4-dihydroxyphenyl)thiazol-2-yl)-2-(4-hydroxymethyl)phenyl)acetamide.

2. Verwendung nach Anspruch 1, **dadurch gekennzeichnet, daß** das oder die Alkylamidothiazole als freie Base oder als Salz vorliegen: z.B. als Fluorid, Chlorid, Bromid, lodid, Sulfat, Carbonat, Ascorbat, Acetat oder Phoshat.

3. Verwendung nach Anspruch 1 oder 2, **dadurch gekennzeichnet, daß** der Gehalt an Alkylamidothiazolen in den kosmetischen Zubereitungen aus dem Bereich von 0,000001 bis 10,0 Gew.-%, insbesondere von 0,0001 bis 3,0 Gew.-% und ganz besonders von 0,001 bis 1 Gew.-% gewählt wird, jeweils bezogen auf das Gesamtgewicht der Zusammensetzung.

4. Alkylamidothiazole nach einem der vorstehenden Ansprüche zur Verwendung bei der medizinischen Prophylaxe und/oder Behandlung von sensibler Haut, Juckreiz sowie von entzündlichen Erscheinungen in der menschlichen Haut.

## Claims

1. Cosmetic use of alkylamidothiazoles in cosmetic preparations for the prophylaxis and treatment of dry skin in human skin, **characterized in that** the alkylamidothiazole(s) have the following structure: 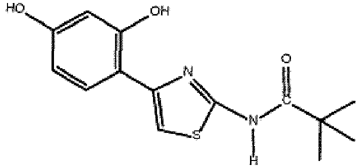 N-(4-(2,4-dihydroxyphenyl)thiazol-2-yl)pivalamide 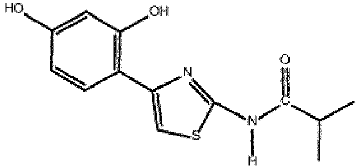 N-(4-(2,4-dihydroxyphenyl)thiazol-2-yl)isobutyramide 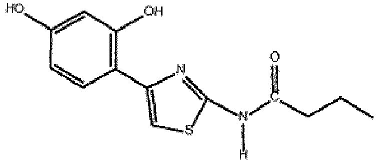 N-(4-(2,4-dihydroxyphenyl)thiazol-2-yl)butyramide 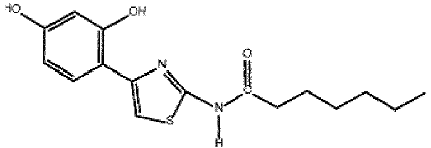 N-(4-(2,4-dihydroxyphenyl)thiazol-2-yl)heptanamide 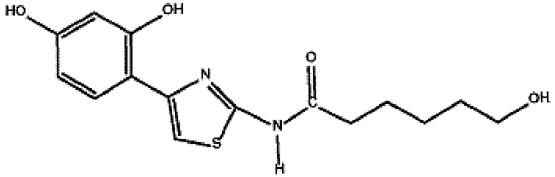 N-(4-(2,4-dihydroxyphenyl)thiazol-2-yl)-6-hydroxyhexanamide 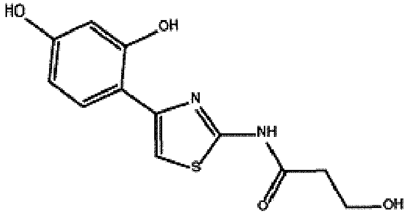 N-(4-(2,4-dihydroxyphenyl)thiazol-2-yl)-3-hydroxypropanamide 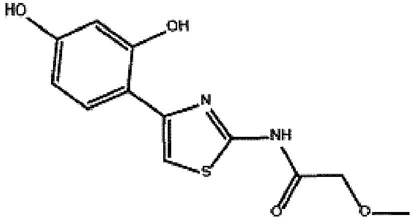 N-(4-(2,4-dihydroxyphenyl)thiazol-2-yl)-2-methoxyacetamide 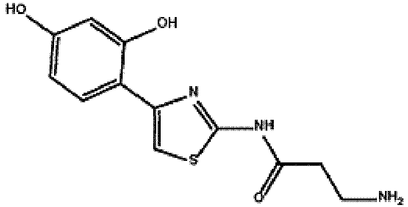 3-amino-N-(4-(2,4-dihydroxyphenyl)thiazol-2-yl)propanamide 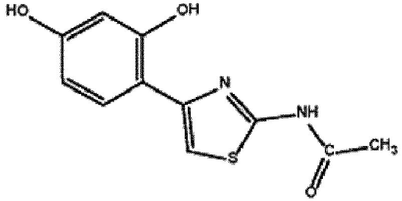 N-(4-(2,4-dihydroxyphenyl)thiazol-2-yl)acetamide 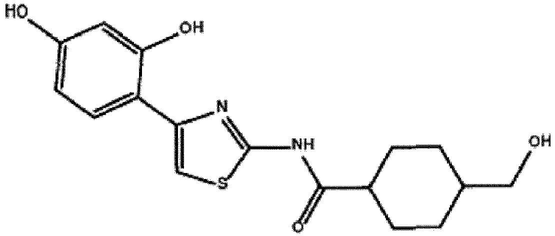 N-(4-(2,4-dihydroxyphenyl)thiazol-2-yl)-4-(hydroxymethyl)cyclohexanecarboxamide 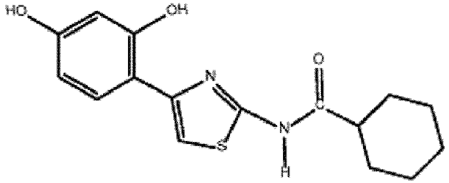 N-(4-(2,4-dihydroxyphenyl)thiazol-2-yl)cyclohexanecarboxamide and 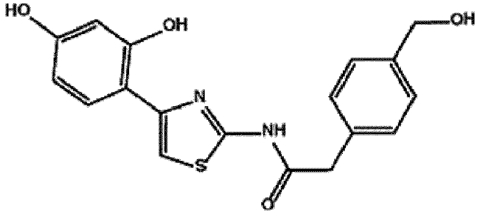 N-(4-(2,4-dihydroxyphenyl)thiazol-2-yl)-2-(4-hydroxymethyl)phenyl)acetamide

2. Use according to Claim 1, **characterized in that** the alkylamidothiazole(s) are present as free bases or as salts: e.g. as fluoride, chloride, bromide, iodide, sulfate, carbonate, ascorbate, acetate or phosphate.

3. Use according to Claim 1 or 2, **characterized in that** the content of alkylamidothiazoles in the cosmetic preparations is selected from the range of 0.000001 to 10.0% by weight, particularly 0.0001 to 3.0% by weight and most particularly from 0.001 to 1% by weight, based in each case on the total weight of the preparation.

4. Alkylamidothiazoles according to any of the preceding claims for use in the medicinal prophylaxis and/or treatment of sensitive skin, pruritis and inflammatory conditions in human skin.

## Revendications

1. Utilisation cosmétique d'alkylamidothiazoles dans des préparations cosmétiques pour la prophylaxie et le traitement de la peau humaine sèche, **caractérisée en ce que** le ou les alkylamidothiazoles présentent la structure suivante : 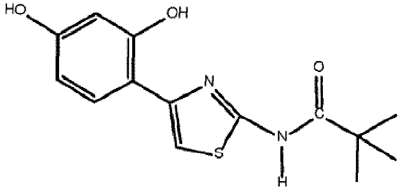 N-(4-(2,4-dihydroxyphényl)thiazol-2-yl)pivalamide 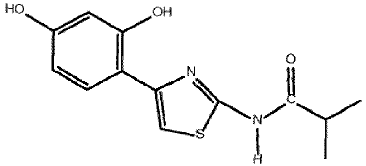 N-(4-(2,4-dihydroxyphényl)thiazol-2-yl)isobutyramide 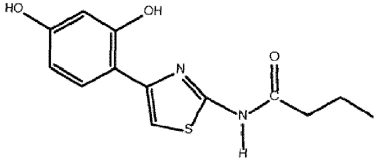 N-(4-(2,4-dihydroxyphényl)thiazol-2-yl)butyramide 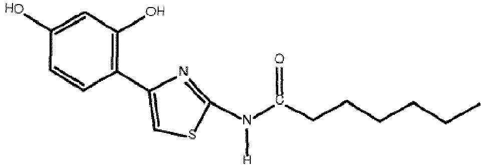 N-(4-(2,4-dihydroxyphényl)thiazol-2-yl)heptanamide 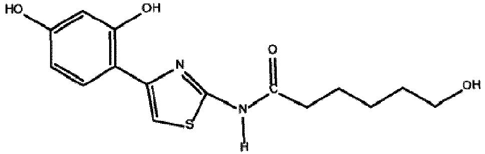 N-(2-(2,4-dihydroxyphényl)thiazol-2-yl)-6-hydroxyhexanamide 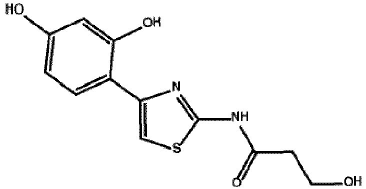 N-(4-(2,4-dihydroxyphényl)thiazol-2-yl)-3-hydroxypropanamide 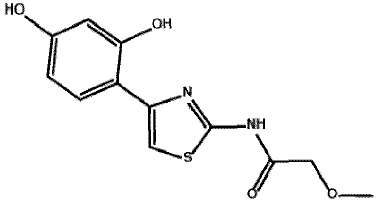 N-(4-(2,4-dihydroxyphényl)thiazol-2-yl)-2-méthoxyacétamide 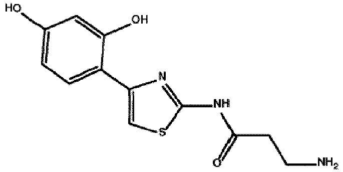 3-amino-N-(4-(2,4-dihydroxyphényl)thiazol-2-yl)propanamide 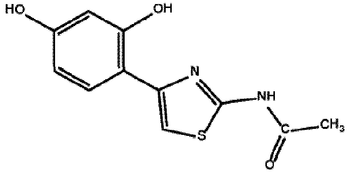 N-(4-(2,4-dihydroxyphényl)thiazol-2-yl)acétamide 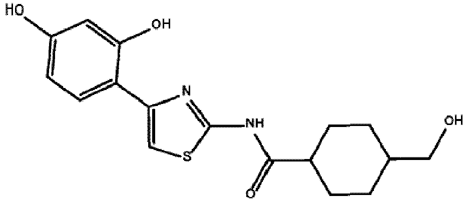 N-(4-(2,4-dihydroxyphényl)thiazol-2-yl)-4-(hydroxyméthyl)cyclohexane-carboxamide 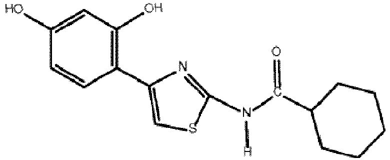 N-(4-(2,4-dihydroxyphényl)thiazol-2-yl)cyclohexane-carboxamide et 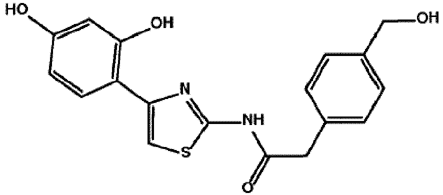 N-(4-(2,4-dihydroxyphényl)thiazol-2-yl)-2-(4-hydroxyméthyl)phényl)acétamide.

2. Utilisation selon la revendication 1, **caractérisée en ce que** le ou les alkylamidothiazoles se présentent sous la forme d'une base libre ou sous la forme d'un sel : p. ex. sous la forme d'un fluorure, d'un chlorure, d'un bromure, d'un iodure, d'un sulfate, d'un carbonate, d'un ascorbate, d'un acétate ou d'un phosphate.

3. Utilisation selon la revendication 1 ou 2, **caractérisée en ce que** la teneur en alkylamidothiazoles dans les préparations cosmétiques est choisie dans la plage allant de 0,000001 à 10,0 % en poids, notamment de 0,0001 à 3,0 % en poids, et tout particulièrement de 0,001 à 1 % en poids, à chaque fois par rapport au poids total de la composition.

4. Alkylamidothiazoles selon l'une quelconque des revendications précédentes, destinés à une utilisation pour la prophylaxie et/ou le traitement médical de la peau sensible, des démangeaisons, ainsi que des phénomènes inflammatoires de la peau humaine.
